# EUROPEAN PATENT SPECIFICATION

(11) **EP 4 486 135 B1**
(45) Date of publication and mention of the grant of the patent: **18.11.2026**
(21) Application number: 22929313.9
(22) Date of filing: 03.03.2022
(51) Int. Cl.: A23G 4/06, A23G 4/20, A61Q 11/00, A61K 8/02, A61K 8/24

(54) **DEEP CLEANING CHEWING GUM**
KAUGUMMI MIT TIEFENREINIGUNG
GOMME À MÂCHER DE NETTOYAGE PROFOND

(43) Date of publication of application: 08.01.2025
(73) Proprietor: WM. Wrigley Jr. Company, Chicago, IL 60642 (US)
(72) Inventor: SUN, Yue, Guangzhou, Guangdong 510730 (CN); YI, Haiquan, Guangzhou, Guangdong 510730 (CN); TIAN, Minmin, Chicago, Illinois 60642 (US); DIAZ, Arturo, Chicago, Illinois 60642 (US); DODDS, Michael, Chicago, Illinois 60642 (US); WINKLER, Kyle, Chicago, Illinois 60642 (US)
(74) Representative: Haseltine Lake Kempner LLP
(86) International application number: PCT/CN2022/078985
(87) International publication number: WO 2023/164863

(56) References cited:
- WO-A1-2020/113184
- CN-A- 101 115 463
- CN-A- 101 203 138
- CN-A- 111 436 520
- CN-A- 112 105 269
- US-A1- 2004 115 247
- US-A1- 2006 263 475
- US-A1- 2009 089 167
- US-B1- 6 685 916
- ARIF BAIG: "Extrinsic whitening effects of sodium hexametaphosphate - a review including a dentifrice with stabilised stannous fluoride", COMPENDIUM CONTIN EDUC DENT VOL.26 NO 9 (SUPPL 1), 1 January 2005 (2005-01-01), pages 47 - 53, XP055326070, Retrieved from the Internet <URL:http://www.dentalcare.es/media/es-ES/research_db/pdf/cphd/baig_whitening.pdf> [retrieved on 20161205]

## Description

### BACKGROUND OF THE DISCLOSURE

The present disclosure is directed to a chewing gum comprising sodium hexametaphosphate that can provide cleaning benefits to teeth, and in particular, that can be used to prevent teeth staining, bacterial attachment, and plaque buildup. Also disclosed are methods for preventing tooth staining using the chewing gum.

US 2004/115247 A1 D1 discloses a dental composition comprising acetic acid or a pharmaceutically acceptable salt thereof and sodium hexametaphosphate, wherein the dental composition is a dental product that can be selected from long a list that comprises chewing gum.

Sodium hexametaphosphate, also known as Glass-H, is a polymeric analogue of pyrophosphate that exhibits strong surface binding characters. Previous studies indicated that Glass-H has a strong affinity to tooth surface. It coats the tooth surface, provides a protecting and shielding layer to tooth enamel, and can prevent the attachment of protein pellicles, food debris and saliva bacteria to the tooth.

Glass-H (GH) can be used in chewing gum and confections at 7.5% or higher concentration. Biesbrock et al. have shown a 7.5% Glass-H gum significantly removed tooth stain vs a control gum (Biesbrock et al, A chewing gum containing 7.5% sodium hexametaphosphate inhibits stain deposition compared with a placebo chewing gum. J Clin Dent 25 253-265 (2004)). Walters et al studied 5.6% sugar free gum (SFG) vs a control gum (Walters et al Benefits of 5.6% SHMP containing chewing gum for extrinsic stain inhibition. J Dent Hygienes 78 (2004)). Porciani et al studied the stain inhibition effect of 4% GH-gum (Porciani, Whitening effect by stain inhibition from SFG with 4% SHMP in a controlled 12-week single blind trial, J Clin Dent 17:14-16 (2006)).

The hydrolytic degradation of condensed polyphosphates is strongly catalyzed by acid (Van Wazer, Chapter 8, Structure and properties of the condensed phosphates in Phosphorus and its compounds Volume 1, Interscience New York 1958). For example, the rate of degradation increases over 10x when pH changes from 6 to 4. For this reason, it was previously recommended that Glass-H has to be formulated into an oral care product with a low moisture level at neutral to basic pH.

Nevertheless, there is a strong consumer desire for sugar-free chewing gum with an enjoyable fruity flavor that helps maintain good oral health condition. Fruity acids such as citric acid, malic acid and lactic acid, are often incorporated in the fruity confections for the desired sensory taste. It would thus be desirable to have a chewing gum that included such fruity acids, while still retaining the stain prevention properties of Glass H.

### SUMMARY OF THE DISCLOSURE

In one aspect, the present disclosure is directed to a chewing gum comprising sodium hexametaphosphate and a fruity acid, wherein the sodium hexametaphosphate is in the gum in an amount of from 0.2 wt% to less than 1 wt%, and wherein the chewing gum comprises from 0.2 to 2.0 wt% of the fruity acid.

In another aspect, the present disclosure is directed to a method for the prevention of tooth staining in a consumer, the method comprising chewing a gum of the present disclosure, wherein the gum provides at least 16.5 mg of sodium hexametaphosphate per serving.

### BRIEF DESCRIPTION OF THE DRAWINGS

Fig. 1A is a graph showing lightness value (L*) of HAP slurry after tea rinse, as described in Example 3.
Fig. 1B is a graph showing whiteness index (WI) of HAP slurry after tea rinse, as described in Example 3.
Fig. 2 is a graph showing the pH of HAP-Glass H (GH) mixtures, with and without citric acid, as described in Example 3.
Fig. 3 is a cyclic staining chamber with blocks of bovine teeth, as described in Example 4.
Fig. 4 is a chewing machine and control unit, as described in Example 4.
Fig. 5 is a graph showing the differences of whiteness index ΔW for various samples after treatment of 7 and 14 days as discussed in Example 4.
Fig. 6 is the calibration curve of PiPer Assay Kit, as described in Example 5.

### DETAILED DESCRIPTION OF THE DISCLOSURE

The present disclosure is directed to a chewing gum comprising sodium hexametaphosphate (Glass H) that can provide cleaning benefits to teeth, and in particular, that can be used to prevent tooth staining, bacterial attachment and plaque buildup. Also disclosed are methods for preventing tooth staining in a consumer using the chewing gum.

Advantageously, it has been found that chewing gum containing low levels of Glass H has a stain preventing effect on teeth. Unexpectedly, this effect is not lost at acidic pH. As such, it has been discovered that Glass H can be incorporated into chewing gum containing fruity flavors with acids, while still maintaining a tooth stain preventive effect.

Thus, in one aspect, the present disclosure is directed to a chewing gum comprising sodium hexametaphosphate (Glass H, or GH), wherein the sodium hexametaphosphate is in the gum in an amount of from about 0.2 wt% to less than about 2 wt%. In another aspect, the chewing gum further comprises a fruity acid.

In another aspect, the present disclosure is directed to a method for the prevention of tooth staining, bacterial attachment and plaque buildup in a consumer, the method comprising chewing a gum of the present disclosure. In one aspect, the gum provides at least 16.5 mg of sodium hexametaphosphate per serving.

### Sodium hexametaphosphate (Glass H)

Sodium hexametaphosphate, also known as Glass-H, is a polymeric analogue of pyrophosphate that exhibits strong surface binding characters. Glass H exhibits a strong affinity to the tooth surface. It coats the tooth, provides a protecting and shielding layer to the tooth enamel, can prevent attachment of protein pellicles, food debris and saliva bacteria to the tooth, and can protect the tooth from staining.

It has now been discovered that this stain preventing effect is maintained when Glass H is incorporated into chewing gum, even at low levels, and in the presence of acids. Thus, in one aspect, the chewing gum of the present disclosure comprises Glass H, wherein the Glass H is in the gum in an amount of less than 1 wt%. In one aspect, the chewing gum comprises from about 0.5 to about 0.8 wt%, from about 0.5 to about 0.7 wt%, or about 0.5 wt% of Glass H.

In one particular embodiment, the Glass-H is encapsulated.

### Fruity acids

The chewing gum of the present disclosure comprises a fruity acid. As used herein, the term "fruity acid" refers to any acid found in fruits or vegetables. Gum formulations, particularly fruit gums, often contain food acids to enhance the flavor perception. Common acids used in gum include citric acid, malic acid, tartaric acid, adipic acid and other acids found in fruits and vegetables.

The chewing gum of the present disclosure comprises from about 0.2 to about 2.0 wt%, or from about 0.3 to about 1.9 wt%, or from about 0.7 to about 1.0 wt% or from about 0.8 to about 0.9 wt%, or from about 0.3 to about 0.5 wt% of the fruity acid.

In one embodiment, the fruity acid is encapsulated.

### Chewing Gum Compositions

The chewing gum compositions of the present disclosure may include Glass H and fruity acids in any of the amounts set forth herein. Chewing gum compositions of the present disclosure may be made using a variety of different compositions that are typically used in chewing gum compositions. Suitable physical forms include sticks, tabs, dragees, chicklets, batons, and the like. Although exact ingredients for each product form will vary from product to product, the specific techniques will be known by one skilled in the art. In general, a chewing gum composition typically contains a chewable gum base portion which is essentially water-insoluble, and a water-soluble bulk portion which includes water soluble bulking agents and other water soluble components as well as flavors and perhaps other active ingredients which are typically water-insoluble. The water-soluble portion dissipates with a portion of the flavor (and other water insoluble actives, if present) over a period of time during chewing. The gum base portion is retained in the mouth throughout the chew.

In one embodiment of the present disclosure, the chewing gum is a coated chewing gum comprising a chewing gum center and a coating. Unless otherwise indicated, the amounts of chewing gum components set forth herein are by weight of the chewing gum center.

The chewing gum of the present disclosure is preferably a coated, sugarless (also referred to herein as sugar free) chewing gum containing the Glass **H,** or Glass H and fruity acids. The chewing gum center may comprise between approximately 5% to about 95% by weight gum base. Typically, the insoluble gum base may comprise between approximately 10% and about 50% by weight of the chewing gum center, or from approximately 20% to about 40% by weight of the chewing gum center. The present disclosure contemplates employing any commercially acceptable gum base.

The insoluble gum base generally comprises elastomers, elastomer solvents, plasticizers, waxes, emulsifiers, and inorganic fillers. Plastic polymers, such as polyvinyl acetate, which behave somewhat as plasticizers, are also included. Other plastic polymers that may be used include polyvinyl laurate, polyvinyl alcohol, and polyvinyl pyrrolidone. Gum base typically comprises 20 to 40% by weight of the overall chewing gum composition. (i.e., by weight of the chewing gum center).

Synthetic elastomers may include, but are not limited to, polyisobutylene (e.g. having a weight average molecular weight of about 10,000 to about 95,000), butyl rubber (isobutylene-isoprene copolymer), styrene copolymers (having for example a styrene-butadiene ratio of about 1:3 to about 3:1), polyisoprene, polyethylene, vinyl acetate-vinyl laurate copolymer (having for example a vinyl laurate content of about 5% to about 50% by weight of the copolymer), and combinations thereof.

Natural elastomers may include for example natural rubbers such as smoke or liquid latex and guayule, as well as natural gums such as chicle, jelutong, lechi caspi, perillo, sorva, massaranduba balata, massaranduba chocolate, nispero, rosindinha, gutta hang kang and mixtures thereof. Preferred elastomers will depend on, for example, whether the chewing gum in which the base is used is adhesive or conventional, synthetic or natural, bubble gum or regular gum. Elastomers provide the rubbery texture which is characteristic of chewing gum. Elastomers typically make up 5 to 25% by weight of the gum base.

Elastomer solvents which are sometimes referred to as elastomer plasticizers, include but are not limited to natural rosin esters such as glycerol esters, or partially hydrogenated rosin, glycerol esters of polymerized rosin, glycerol esters of partially dimerized rosin, glycerol esters of rosin, pentaerythritol esters of partially hydrogenated rosin, methyl and partially hydrogenated methyl esters of rosin, pentaerythritol esters of rosin, synthetics such as terpene resins, polylimonene and other polyterpenes and/or any suitable combination of the forgoing. Elastomer solvents are typically employed at levels of 5 to 30% by weight of the gum base.

Gum base plasticizers are sometimes referred to as softeners (but are not to be confused with water soluble softeners used in the water soluble portion of the gum). Typically, these include fats and oils as well as waxes. Fats and oils are typically vegetable oils which are usually partially or fully hydrogenated to increase their melting point. Vegetable oils suitable for such use include oils of cottonseed, soybean, palm (including palm kernal), coconut, shea, castor, peanut, corn, rapeseed, canola, sunflower, cocoa and others. Less commonly used are animal fats such as milk fat, tallow and lard. Structured fats, which are essentially synthetically compounded glycerol esters (triglycerides) of fatty acids of varying chain lengths, offer an ability to carefully adjust the softening profile by use of short and medium chain fatty acids which are less commonly found in nature. Commonly employed waxes include paraffin, microcrystalline and natural waxes such as beeswax and carnauba. Microcrystalline waxes, especially those with a high degree of crystallinity, may be considered bodying agents or textural modifiers. Plasticizers are typically employed at a level of 5 to 40% by weight of the gum base.

Plastic polymers, such as polyvinyl acetate, which behave somewhat as plasticizers, are also commonly used. Other plastic polymers that may be used include polyvinyl laurate, polyvinyl alcohol, and polyvinyl pyrrolidone. Most gum bases incorporate polyvinyl acetate at a level of 5 to 40% by weight of the gum base.

The gum base typically also includes a filler component. The filler component is typically an inorganic powder such as calcium carbonate, ground limestone, magnesium carbonate, talc, silicate types such as aluminum and magnesium silicate, dicalcium phosphate, tricalcium phosphate, cellulose polymers, such as wood, combinations thereof and the like. The filler may constitute from 5% to about 50% by weight of the gum base. Occasionally, a portion of the filler may be added to the chewing gum mixture separately from the gum base.

Emulsifiers, which may also have plasticizing properties, assist in homogenizing and compatibilizing the different base components. Commonly used emulsifiers include mono- and diglycerides such as glycerol monostearate, lecithin, glycerol triacetate, glycerol monostearate, acetylated monoglycerides, fatty acids and combinations thereof. Emulsifiers are commonly used at a level of 1 to 10% by weight of the gum base.

Gum bases commonly contain optional additives such as other antioxidants and colors which serve their normal functions. Less commonly, flavors and sweeteners may be added to the gum base. These additives, if used, are typically employed at levels of about 1% or less by weight of the gum base.

The water-soluble portion of the chewing gum center may comprise softeners, sweeteners, flavoring agents, and combinations thereof as well as other optional ingredients. For example, the majority of the water soluble portion of the chewing gum center will typically comprise a water-soluble, powdered carbohydrate which serves as a bulking agent. In sugar gums, this most often is sucrose although other sugars such as fructose, erythrose, dextrose (glucose), levulose, tagatose, galactose, trehalose, corn syrup solids and the like, alone or in any combination may also be used.

Generally, sugarless chewing gums will employ sugar alcohols (also called alditols, polyols or polyhydric alcohols) as bulking agents due to their benefits of low cariogenicity, reduced caloric content and reduced glycemic values. Such sugar alcohols include sorbitol, mannitol, xylitol, hydrogenated isomaltulose, maltitol, erythritol, hydrogenated starch hydrolysate solids, and the like, alone or in any combination. Longer chain saccharides such as polydextrose and fructo-oligosaccharides are sometimes employed for their reduced caloric properties and other health benefits. The bulking agents typically comprise approximately 5% to about 95% by weight of the chewing gum center.

Softeners are added to the chewing gum in order to optimize the chewability and mouth feel of the gum. Softeners, also known in the art as plasticizers or plasticizing agents, generally constitute between approximately 0.5% to about 15% by weight of the chewing gum center. These include glycerin, propylene glycol and aqueous sweetener solutions (syrups). Examples of syrups include corn syrups and (generically) glucose syrups which are usually prepared from hydrolyzed starch. For sugarless products, the starch hydrolysate may be hydrogenated to produce an ingredient known as hydrogenated starch hydrolysate syrups or maltitol syrups. These HSH syrups have largely replaced sorbitol solutions previously used in sugarless gums because they also function as binders to improve the flexibility and other physical properties of the gum. Softeners are also often used to control the humectancy (water absorbing properties) of the product.

An emulsifier is sometimes added to the gum to improve the consistency and stability of the gum product. They may also contribute to product softness. Lecithin is the most commonly employed emulsifier, although nonionic emulsifiers such as polyoxyethylene sorbitan fatty acid esters and partial esters of common fatty acids (lauric, palmitic, stearic and oleic acid hexitol anhydrides) derived from sorbitol may also be used. When used, emulsifiers typically comprised 0.5 to 2% by weight of the chewing gum center.

Suitable surface active agents can be salts of potassium, ammonium, or sodium. Sodium salts include anionic surface active agents, such as alkyl sulfates, including sodium lauryl sulfate, sodium laureth sulfate, and the like. Other sodium salts include sodium lauroyl sarcosinate, sodium brasslate, and the like. Suitable ammonium salts include betaine derivatives such as cocamidopropyl betaine, and the like.

In the case of sugarless gums, it is usually desirable to add high intensity sweeteners to compensate for the reduced sweetness resulting from substitution of sugar alcohols for the sucrose in sugar gums. More recently, the trend has been to also add high intensity sweeteners to sugar gums to boost and extend flavor and sweetness. High intensity sweeteners (which are sometimes called high potency or artificial sweeteners) may be defined as food acceptable chemicals which are at least twenty times sweeter than sucrose. Commonly used high intensity sweeteners include aspartame, sucralose, and acesulfame-K. Less common are saccharin, thaumatin, alitame, neotame, cyclamate, perilla derived sweeteners, stevia derived sweeteners, monatin, monellin and chalcones.

Usage levels for high intensity sweeteners may vary widely depending on the potency of the sweetener, local market preferences and the nature and level of other ingredients which might impart bitterness to the gum. Typical levels can range from about 0.01% to about 2% by weight, although some applications may dictate usage outside that range. These sweeteners may be combined together, or with non-high intensity sweeteners at varying levels to impart a sweetness synergy to the overall composition.

Flavors can also optionally be employed to impart a characteristic aroma and taste sensation to chewing gum products. The chewing gum and methods described herein are not limited in this regard. As discussed herein, most flavors are water insoluble liquids, but water soluble liquids and solids are also known. These flavors may be natural or artificial (synthetic) in origin. Often natural and artificial flavors are combined. It is also common to blend different flavors together in pleasing combinations. Although the range of flavors usable in chewing gums is nearly limitless, they commonly fall into several broad categories. Fruit flavors include lemon, orange, lime, grapefruit, tangerine, strawberry, apple, cherry, raspberry, blackberry, blueberry, banana, pineapple, cantaloupe, muskmelon, watermelon, grape, currant, mango, kiwi and many others as well as combinations. Mint flavors include spearmint, peppermint, wintergreen, basil, corn mint, menthol and others and mixtures thereof. Spice flavors include cinnamon, vanilla, clove, chocolate, nutmeg, coffee, licorice, eucalyptus, ginger, cardamom and many others. Also used are herbal and savory flavors such as popcorn, chili, corn chip and the like. Flavors are typically employed at levels of 0.1 to 4% by weight of the finished gum product (e.g., coated chewing gum). In some embodiments, increased flavor levels may be employed to provide a higher flavor impact.

It is common to co-dry and encapsulate flavors with various carriers and/or diluents. For example, spray-dried flavors using gum Arabic, starch, cyclodextrin or other carriers are often used in chewing gum for protection, controlled release, control of product texture and easier handling as well as other reasons. When flavors are in such forms, it will often be necessary to increase the usage level to compensate for the presence of the carriers or diluents.

The chewing gum of the present disclosure may employ various sensates. Generally, sensates may be any compounds that cause a cooling, heating, warming, tingling or numbing, for example, to the mouth or skin. Cooling agents are trigeminal stimulants that impart a cool sensation to the mouth, throat and nasal passages. The most widely known cooling agent is menthol, although this is often considered a flavor due to its aroma properties and the fact that it is a natural component of peppermint oil. More often, the term cooling agent refers to other natural or synthetic chemicals used to impart a cooling sensation with minimal aroma. Commonly employed cooling agents include ethyl p-menthane carboxamide and other N-substituted p-menthane carboxamides, N,2,3-trimethyl-2-isopropyl-butanamide and other acyclic carboxamides, menthyl glutarate (Flavor Extract Manufacturing Association (FEMA 4006)), 3-1-menthoxypropane-1,2-diol, isopulegol, menthyl succinate, menthol propylene glycol carbonate, menthol ethylene glycol carbonate, menthyl lactate, menthyl glutarate, menthone glyceryl ketal, p-menthane-1,8-diol, menthol glyceryl ether, N-tertbutyl-p-menthane-3-carboxamide, p-menthane-3-carboxylic acid glycerol ester, methyl-2-isopryl-bicyclo (2.2.1), heptane-2-carboxamide, menthol methyl ether and others and combinations thereof.

Cooling agents may be employed to enhance the cool taste of mint flavors or to add coolness to fruit and spice flavors. Cooling agents also provide the perception of breath freshening, which is the basis of the marketing of many chewing gums and confections.

Trigeminal stimulants other than cooling agents may be employed in the chewing gums of the present disclosure. These include warming agents such as capsaicin, capsicum oleoresin, red pepper oleoresin, black pepper oleoresin, piperine, ginger oleoresin, gingerol, shoagol, cinnamon oleoresin, cassia oleoresin, cinnamic aldehyde, eugenol, cyclic acetal of vanillin, menthol glycerin ether and unsaturated amides and tingling agents such as Jambu extract, vanillyl alkyl ethers such as vanillyl n-butyl ether, spilanthol, Echinacea extract and Northern Prickly Ash extract. Some of these components are also used as flavoring agents.

Chewing gum generally conveys oral care benefits. In addition to mechanical cleaning of the teeth provided by the chewing action, saliva stimulated by chewing, flavor and taste from the product conveys additional beneficial properties in reducing bad breath, neutralizing acid, and the like. Saliva also contains beneficial polypeptides and other components which may improve the oral environment. These include antimicrobial proteins such as lysozyme, lactoferrin, peroxidases, and histatins; and inhibitors of spontaneous crystallization, such as statherin.

The chewing gums of the present disclosure can provide these benefits along with the benefits disclosed herein, and may also be used as vehicles for the delivery of specialized oral care agents. These may include antimicrobial compounds such as Cetylpyridinium Chloride (CPC), triclosan and chlorhexidine; anti-caries agents such as calcium and phosphate ions, plaque removal agents such as abrasives, surfactants and compound/ingredients; plaque neutralization agents such as ammonium salts, urea and other amines; anti-tartar/calculus agents such as soluble pyrophosphates salts; anti halitosis agents such as parsley oil and copper or zinc salts of gluconic acid, lactic acid, acetic acid or citric acid, and whitening agents such as peroxides; agents that may provide either local or systemic anti-inflammatory effects to limit gingivitis, such as COX-2 inhibitors; agents that may reduce dentinal hypersensitivity, such as potassium salts to inhibit nerve cell transmission; and calcium phosphate salts to block the dentinal tubules.

Certain flavors such as peppermint, methyl salicylate, thymol, eucalyptol, cinnamic aldehyde and clove oil (eugenol) may have antimicrobial properties which benefit the oral cavity. These flavors may be present primarily for flavoring purposes or may be added specifically for their antimicrobial properties.

Certain mineral agents may contribute to dental health, by combating demineralization and enhancing remineralization of teeth. Such ingredients include fluoride salts, dental abrasives and combinations thereof.

Teeth color modifying substances may be considered among the oral care actives useful. These substances are suitable for modifying the color of the teeth to satisfy the consumer such as those listed in the CTFA Cosmetic Ingredient Handbook, 3rd Edition, Cosmetic and Fragrances Associations Inc., Wash. D.C. (1982), incorporated herein by reference. Specific examples include talc, mica, magnesium carbonate, magnesium silicate, aluminum magnesium carbonate, silica, titanium dioxide, zinc oxide, red iron oxide, brown iron oxide, yellow iron oxide, black iron oxide, ferric ammonium ferrocyanide, manganese violet, ultramarine, nylon powder, polyethylene powder and mixtures thereof.

The chewing gums of the present disclosure may be used to deliver biologically active agents and other beauty ingredients to the chewer. Such ingredients include vitamins, minerals, anti-oxidants, nutritional supplements, dietary supplements, functional food ingredients (e.g., probiotics, prebiotics, lycopene, phytosterols, stanol/sterol esters, omega-3 fatty acids, adenosine, lutein, zeaxanthin, grape seed extract, ginkgo biloba, isothiocyanates and the like), OTC and prescription pharmaceuticals, vaccines, and nutritional supplements.

It may be desirable to take certain steps to increase or decrease the rate of the release of the agent or to ensure that at least a minimum quantity is released. Such measures as encapsulation, isolation of the active, measures to increase or decrease interaction with the water-insoluble portion of the gum and enteric coating of actives may be employed to that end.

Thus in one aspect, the chewing gum of the present disclosure comprises encapsulated fruity acids. The acids may be encapsulated using any technique known in the art. Suitable encapsulated acids are also commercially available, e.g., from Walton company (https://www.waltonsinc.com/encapsulated-citric-acid). Without wishing to be bound to any particular theory, it is believed that encapsulating the acids may help improve Glass-H stability and shelf life of the chewing gum.

In another aspect, the chewing gum of the present disclosure may further comprise encapsulated Glass-H. Without wishing to be bound to any particular theory, it is believed that encapsulating the Glass-H blocks the Glass H from reacting with external factors known to initiate and continue degradation, thus enhancing the stability and shelf-life of the chewing gum. The Glass-H may be encapsulated using any known technique. **In** one embodiment, the encapsulant is available from Armando Castro (80% encapsulation with gum acacia).

In general, chewing gum is manufactured by sequentially adding the various chewing gum ingredients to a commercially available mixer known in the art. After the ingredients have been thoroughly mixed, the gum mass is discharged from the mixer and shaped into the desired form such as rolling sheets and cutting into sticks, extruding into chunks or casting into pellets, which are then coated or panned. A pellet center may be coated with a hard shell coating that may also contain flavoring agents to give a fast release of flavor initially. Alternatively, or in addition, a liquid center fill may be coextruded with the gum mass to produce a center filled product.

Generally, the ingredients are mixed by first melting the gum base and adding it to the running mixer. The base may also be melted in the mixer itself. Color or emulsifiers may also be added at this time. A softener such as glycerin may also be added at this time, along with syrup and a portion of the bulking agent. Further parts of the bulking agent are added to the mixer. The Glass H and/or fruity acids may be added with the bulking agent and/or with any high intensity sweeteners. Other optional ingredients are added to the batch in a typical fashion, well known to those of ordinary skill in the art.

The entire mixing procedure typically takes from five to fifteen minutes, but longer mixing times may sometimes be required. Those skilled in the art will recognize that many variations of the above described procedure may be followed.

Chewing gum base and chewing gum product have been manufactured conventionally using separate mixers, different mixing technologies and, often, at different factories. One reason for this is that the optimum conditions for manufacturing gum base, and for manufacturing chewing gum from gum base and other ingredients such as sweeteners and flavors, are so different that it has been impractical to integrate both tasks. Chewing gum base manufacturing involves the dispersive (often high shear) mixing of difficult-to-blend ingredients, such as elastomer, filler, elastomer plasticizer, base softeners/emulsifiers, and sometimes waxes. This process typically requires long mixing times. Chewing gum product manufacture also involves combining the gum base with more delicate ingredients such as product softeners, bulk sweeteners, high intensity sweeteners and flavoring agents using distributive (generally lower shear) mixing, for shorter periods.

In some embodiments, chewing gums of the present invention are also coated. For example, a chewing gum as described herein may be formed into pellets that are pillow shaped, or into balls. The pellets/balls can be then sugar coated or panned by conventional panning techniques to make a unique sugar coated pellet gum.

Conventional panning procedures generally coat with sucrose, but recent advances in panning have allowed the use of other carbohydrate materials to be used in the place of sucrose. Some of these components include, but are not limited to, dextrose, maltose, palatinose, xylitol, lactitol, hydrogenated isomaltulose and other new alditols or a combination thereof. These materials may be blended with panning modifiers including, but not limited to, gum arabic, maltodextrins, corn syrup, gelatin, cellulose type materials like carboxymethyl cellulose or hydroxymethyl cellulose, starch and modified starches, vegetable gums like alginates, locust bean gum, guar gum, and gum tragacanth, insoluble carbonates like calcium carbonate or magnesium carbonate and talc. Antitack agents may also be added as panning modifiers which allow the use of a variety of carbohydrates and sugar alcohols to be used in the development of new panned or coated gum products. Essential oils may also be added with the sugar coating to yield unique product characteristics.

In one embodiment, the chewing gum is a center filled chewing gum. In particular, the chewing gum may be a liquid center filled gum or a powdered center filled gum. For example, the chewing gum may be center-filled with a liquid, syrup, or powder. The center filling may contain vitamins, supplements, nutritional ingredients, minerals, herbal extracts, oligosaccharides and the like. Such center filling could also include chocolate and other forms of confectionery products. In one embodiment, the chewing gum may also contain a high intensity sweetener, natural or artificial sweetener, sugar alcohol, or other sugar substitute in place of all or part of its sucrose or glucose syrup. Methods for preparing center-filled chewing gums are described in US 7,771 182.

The chewing gum of the present disclosure may have any desired shape, including, but not limited to, ball, lentil, cube, and pellet. In one embodiment, the chewing gum has a diameter of from about 11 mm to about 19 mm.

### Methods of Preventing Tooth Staining

In another aspect, the present disclosure is directed to a method for the prevention of tooth staining in a consumer, comprising chewing a gum of the present disclosure. In one particular aspect, the gum is chewed for at least 10 minutes, or for at least 12 minutes. In another aspect, the gum is chewed at least 1 time per day, at least 2 times per day, or at least 3 times per day. In one embodiment, the gum is chewed 1 to 3 times per day for at least 7 days. In another embodiment, the gum is chewed 1 to 3 times per day for at least 14 days.

In one aspect, the chewing gum used in the methods of the present disclosure provides at least 16.5 mg of Glass H per serving. In certain embodiments, a serving may comprise 1, 1.5, 2 or more individual pieces of chewing gum.

The following examples are illustrative of preferred embodiments of the disclosure and are not to be construed as limiting. All percentages are based on the percent by weight of the composition unless otherwise indicated, and all totals equal 100% by weight.

### EXAMPLES

In all the examples below chewing gums comprising sodium hexametaphosphate (Glass H) outside the range of from 0.2 wt% to less than 1 wt%, and the fruity acid outside the range of from 0.2 to 2.0 wt% are not part of the present invention.

### Example 1: Chewing gum formulation

A chewing gum comprising Glass H was formulated containing the ingredients in Table A.

**Table A**

| **Description** | **Pct(%)** |
|---|---|
| Xylitol | 46.0-48.0 |
| Gum base | 27.0-30.0 |
| SORBITOL | 6.5-7.0 |
| Citric Acid | 0.1-0.6 |
| Malic Acid | 0.1-0.4 |
| Menthol | 0.0-0.2 |
| High intense sweetener | 0.5-0.7 |
| Glass-H | 0.2-0.5 |
| XANTHAN | 0.004 |
| Colorant | 0.002 |
| Rice starch | 2.4 |
| Flavor | 0.5-2.0 |
| Maltodextrin | 1.4-1.6 |
| Mannitol | 0.9-1.2 |
| Gum acacia | 0.3-0.5 |
| SPECKLE(optional) | 0.0-0.05 |
| Talc | 0.03-0.05 |
| Carnauba Wax | 0.03-0.05 |
| 75% Maltitol syrup | 7.0-9.0 |
| Process Water | 0.04-0.1 |

### Example 2: Chewing gum formulations

Various chewing gums comprising Glass H were formulated containing the ingredients set forth below.

### Sample 1 - Liquid center filled chewing gum

A coated sugar free liquid center filled chewing gum comprising 85 wt% gum formulation and 15 wt% liquid filling was prepared. The coating comprised 74 wt% dusting layer, and 26 wt% coating layer. The gum formulation components are set forth below in Table B1, the liquid filling components are set forth below in Table B2, and the coating components are set forth below in Table B3.

**Table B1 - gum formulation**

| **Description** | **Pct(%)** |
|---|---|
| Xylitol | 36.0-38.0 |
| Gum base | 43.0-46.0 |
| SORBITOL | 11.0-13.0 |
| Flavor | 2.0-3.0 |
| Citric Acid | 0.8-1.2 |
| Malic Acid | 0.6-1.0 |
| Menthol | 0.4-0.6 |
| High intense sweetener | 0.8-1.2 |
| Artificial Cooling Flavor Enhancer | 0.1-0.2 |
| Glass-H | 0.8 |

**Table B2 - Liquid filling**

| Item Name | | Pct(%) |
|---|---|---|
| MAL 75/HLX Preblend^{c} | 75% Maltitol syrup mixed with Xylitol | 98.3-98.9 |
| 10% XANTHAN GUM SOL. 10%a | XANTHAN syrup | 0.3-0.5 |
| 10% BBLU Solution^{b} | Blue dye | 0.01-0.03 |
| Cooling agent | | 0.8-1.2 |

| | | |
|---|---|---|
| ^{a} 90% water, 10% xanthan gum ^{b}90% water, 10% blue dye ^{c}71.2% of 75% maltitol syrup; 28.8% xylitol | | |

**Table B3 - Coating**

| **Coating Layers** | |
|---|---|
| Item Name | Pct(%) |
| Syrup 1 | 68 |
| Syrup 2 | 28 |
| Flavor | 2.3 |
| Speckle (color) (optional) | 0.0-1.4 |
| Talc | 0.18-0.22 |
| Wax | 0.10-0.15 |

### Syrup 1

| Item Name | Pct(%) |
|---|---|
| Xylitol | 64 |
| Water | 20 |
| Rice starch | 7 |
| Maltodextrin | 6 |
| Mannitol | 3 |

### SFL Syrup 2

| Item Name | Pct(%) |
|---|---|
| Xylitol | 63 |
| Water | 19 |
| Gum Acacia Solution 40% | 8 |
| Rice starch | 7 |
| Mannitol | 3 |

The chewing gums were formulated into 2 g balls having a diameter of 14.5 mm.

### Sample 2 - Powder filled chewing gum

A coated sugar free powder center filled chewing gum comprising 85 wt% gum formulation and 15 wt% powder filling was prepared. The coating comprised 74 wt% dusting layer, and 26 wt% coating layer. The gum formulation components are set forth below in Table C1, the powder filling components are set forth below in Table C2, and the coating components are set forth below in Table C3.

### Table C1 - Gum Formulation

| **Description** | **Pct(%)** |
|---|---|
| Xylitol | 36.0-38.0 |
| Gum base | 43.0-46.0 |
| Sorbitol | 11.0-13.0 |
| Flavor | 2.7-3.0 |
| Citric Acid (Anhydrous) | 0.8-1.20 |
| Malic Acid | 0.6-1.0 |
| Menthol | 0.5 |
| High intense sweetener | 0.8-1.2 |
| Artificial Cooling Flavor Enhancer | 0.1-0.2 |
| Glass-H | 0.8 |

**Table C2 - Powder filling**

| Item name | Pct(%) |
|---|---|
| SORBITOL | 45.0-55.0 |
| Citric Acid (Anhydrous) | 25.0-29.0 |
| Baking soda | 19.0-25.0 |
| Artificial Cooling Flavor Enhancer | 0.5-1.5 |

**Table C3 - Coating**

| **Coating Layers** | |
|---|---|
| Item Name | Pct(%) |
| Syrup 1 | 67.7 |
| Syrup 2 | 28 |
| Flavor | 2.0-3.0 |
| Speckle (Color) (optional) | 0-1.4 |
| Talc | 0.2 |
| Carnauba Wax | 0.1 |

### Syrup 1

| Item name | Pct(%) |
|---|---|
| Xylitol | 64 |
| Water | 20 |
| Rice starch | 7 |
| Maltodextrin | 6 |
| Mannitol | 3 |

### Syrup 2

| Item name | Pct(%) |
|---|---|
| Xylitol | 63 |
| Water | 19 |
| Gum acacia 40% | 8 |
| Rice starch | 7 |
| Mannitol | 3 |

The chewing gums were formulated into 2 g balls having a diameter of 14.5 mm.

### Sample 3 - Color Change Chewing Gum

A coated color change chewing gum was prepared. The coating comprised 70 wt% dusting layer, and 30 wt% coating layer. The gum formulation components are set forth below in Table D1, and the coating components are set forth below in Table D2.

**Table D1 - Gum Formulation**

| **Description** | **Pct(%)** |
|---|---|
| Xylitol | 40.0-45.0 |
| Gum base | 31.0-37.0 |
| Sorbitol | 11.0-13.0 |
| Maltitol | 4.0-6.0 |
| Flavor | 2.0-3.0 |
| Citric Acid (Anhydrous) | 0.8-1.2 |
| Malic Acid | 0.6-1.0 |
| Menthol | 0.3-0.5 |
| High intense sweetener | 1.0-1.3 |
| Artificial Cooling Flavor Enhancer | 0.2-0.4 |
| FD&C Blue No. 1 | 0.08-0.12 |
| Glass-H | 0.7 |

**Table D2 - Coating**

| **Coating Layers** | |
|---|---|
| Item Name | Pct(%) |
| Syrup 1 | 67 |
| Syrup 2 | 29 |
| Flavor | 2.0-3.0 |
| Speckle (Color) (optional) | 0-1.1 |
| Talc | 0.2 |
| Carnauba Wax | 0.15 |

### Syrup 1

| Item name | Pct(%) |
|---|---|
| Xylitol | 64 |
| Water | 20 |
| Rice starch | 7 |
| Maltodextrin | 6 |
| Mannitol | 3 |

### Syrup 2

| Item name | Pct(%) |
|---|---|
| Xylitol | 63 |
| Water | 19 |
| Gum acacia 40% | 8 |
| Rice starch | 7 |
| Mannitol | 3 |

The chewing gums were formulated into 1.5 g lentil shaped gums having a diameter of 12 mm.

### Sample 4 - Flavor Beads

Coated flavored bead chewing gums were prepared. The coating comprised 70 wt% dusting layer, and 30 wt% coating layer. The gum formulation components are set forth below in Table E1, and the coating components are set forth below in Table E2.

**Table E1 - Gum formulation**

| **Description** | **Pct(%)** |
|---|---|
| Xylitol | 44.0-47.0 |
| Gum base | 33.5-35.5 |
| Sorbitol | 11.0-13.0 |
| Flavor | 2.0-3.0 |
| Citric Acid | 0.8-1.2 |
| Malic Acid | 0.6-1.0 |
| Menthol | 0.5 |
| High intense sweetener | 0.8-1.2 |
| Artificial Cooling Flavor Enhancer | 0.15 |
| Encapsulated flavor | 0.6-1.0 |
| Glass-H | 0.714 |

**Table E2 - Coating**

| **Coating Layers** | |
|---|---|
| Item Name | Pct(%) |
| Syrup 1 | 66.7 |
| Syrup 2 | 29 |
| Flavor | 2.0-3.0 |
| Speckle (Color) (optional) | 0-1.4 |
| Talc | 0.18 |
| Carnauba Wax | 0.15 |

### Syrup 1

| Item name | Pct(%) |
|---|---|
| Xylitol | 64 |
| Water | 20 |
| Rice starch | 7 |
| Maltodextrin | 6 |
| Mannitol | 3 |

### Syrup 2

| Item name | Pct(%) |
|---|---|
| Xylitol | 63 |
| Water | 19 |
| GUM TAHLA Solution 40% (60% water, 40% acacia powder) | 8 |
| Rice starch | 7 |
| Mannitol | 3 |

The chewing gums were formulated into 1.4 g cube shaped beads having a diameter of 15 mm.

### Sample 5 - Coated chewing gum

Coated chewing gums were prepared. The coating comprised 70 wt% dusting layer, and 30 wt% coating layer. The gum formulation components are set forth below in Table F1, and the coating components are set forth below in Table F2.

**Table F1 - gum formulation**

| **Description** | **Pct(%)** |
|---|---|
| Xylitol | 41.0-43.0 |
| Gum base | 33.5-35.0 |
| Sorbitol | 11.0-13.0 |
| Maltitol | 4.5-5.5 |
| Flavor | 2.0-3.0 |
| Citric Acid | 0.8-1.2 |
| Malic Acid | 0.6-1.0 |
| Menthol | 0.5 |
| High intense sweetener | 0.8-1.2 |
| Artificial Cooling Flavor Enhancer | 0.3-0.5 |
| Glass-H | 0.714 |

**Table F2 - Coating**

| **Coating Layers** | |
|---|---|
| Item Name | Pct(%) |
| Syrup 1 | 66.7 |
| Syrup 2 | 29 |
| Flavor | 2.0-3.0 |
| Speckle (optional) | 0-1.4 |
| Talc | 0.18 |
| Carnauba Wax | 0.15 |

### Syrup 1

| Item name | Pct(%) |
|---|---|
| Xylitol | 64 |
| Water | 20 |
| Rice starch | 7 |
| Maltodextrin | 6 |
| Mannitol | 3 |

### Syrup 2

| Item name | Pct(%) |
|---|---|
| Xylitol | 63 |
| Water | 19 |
| Gum Acacia Solution 40% | 8 |
| Rice starch | 7 |
| Mannitol | 3 |

The chewing gums were formulated into 1.5 g cube shaped gums having a diameter of 11-19 mm.

### Sample 6 - Coated Chewing Gum

Coated chewing gums were prepared. The coating comprised 70 wt% dusting layer, and 30 wt% coating layer. The gum formulation components are set forth below in Table G1, and the coating components are set forth below in Table G2.

**Table G1 - gum formulations**

| **Description** | **Pct(%)** |
|---|---|
| Xylitol | 40.0-44.0 |
| Gum base | 32.0-36.0 |
| Sorbitol | 11.0-13.0 |
| Maltitol | 4.5-5.5 |
| Flavor | 2.0-2.6 |
| Citric Acid (Anhydrous) | 0.9-1.3 |
| Malic Acid | 0.6-1.0 |
| Menthol | 0.5 |
| High intense sweetener | 0.8-1.1 |
| Artificial Cooling Flavor Enhancer | 0.3 |
| Glass-H | 0.714 |

**Table G2 - Coating**

| **Coating Layers** | |
|---|---|
| Item Name | Pct(%) |
| Syrup 1 | 66.7 |
| Syrup 2 | 29 |
| Flavor | 2.2-3.0 |
| Speckle (Color) (optional) | 0-1.4 |
| Talc | 0.18 |
| Carnauba Wax | 0.15 |

### Syrup 1

| Item name | Pct(%) |
|---|---|
| Xylitol | 64 |
| Water | 20 |
| Rice starch | 7 |
| Maltodextrin | 6 |
| Mannitol | 3 |

### Syrup 2

| Item name | Pct(%) |
|---|---|
| Xylitol | 63 |
| Water | 19 |
| Acacia solution 40% | 8 |
| Rice starch | 7 |
| Mannitol | 3 |

The chewing gums were formulated into 1.5 g cube shaped gums having a diameter of 11-19 mm.

### Sample 7 - Coated Chewing Gum

Coated chewing gums were prepared. The coating comprised 70 wt% dusting layer, and 30 wt% coating layer. The gum formulation components are set forth below in Table H1, and the coating components are set forth below in Table H2.

**Table H1 - Gum formulation**

| **Description** | **Pct(%)** |
|---|---|
| Xylitol | 41.0-44.0 |
| Gum base | 32.0-36.0 |
| Sorbitol | 11.0-13.0 |
| Maltitol | 4.0-6.0 |
| Flavor | 1.8-2.5 |
| Citric Acid | 0.9-1.1 |
| Malic Acid | 0.7-0.9 |
| Menthol | 0.5 |
| High intense sweetener | 0.8-1.1 |
| Artificial Cooling Flavor Enhancer | 0.3-0.5 |
| Glass-H | 0.714 |

**Table H2 - Coating**

| **Coating Layers** | |
|---|---|
| Item Name | Pct(%) |
| Syrup 1 | 66.7 |
| Syrup 2 | 29 |
| Flavor | 2.0-2.6 |
| Speckle (Color) (optional) | 0-1.4 |
| Talc | 0.18 |
| Carnauba Wax | 0.15 |

### Syrup 1

| Item name | Pct(%) |
|---|---|
| Xylitol | 64 |
| Water | 20 |
| Rice starch | 7 |
| Maltodextrin | 6 |
| Mannitol | 3 |

100

### Syrup 2

| Item name | Pct(%) |
|---|---|
| Xylitol | 63 |
| Water | 19 |
| GUM TAHLA Solution 40% | 8 |
| Rice starch | 7 |
| Mannitol | 3 |

The chewing gums were formulated into 1.4 - 1.5 g cube shaped gums having a diameter of 11-19 mm.

### Sample 8 - Coated Chewing Gum

Coated chewing gums were prepared. The coating comprised 80 wt% dusting layer, and 20 wt% coating layer. The gum formulation components are set forth below in Table I1, and the coating components are set forth below in Table I2.

**Table I1 - Gum formulation**

| **Description** | **Pct(%)** |
|---|---|
| Xylitol | 42.7 |
| Sorbitol | 25 |
| Gum base -1 | 16.2 |
| Gum base -2 | 10.8 |
| Neat and encapsulated high intense sweetener | 1.2 |
| Mouthwatering agent | 0.5 |
| WS-23 | 0.15 |
| Fruit Acid | 1.8 |
| Acacia | 1 |
| Glass-H | 0.625 |
| Total | 100 |

**Table I2 - Coating**

| **Coating Layers** | |
|---|---|
| Item Name | Pct(%) |
| SYRUP 1 | 99.7 |
| Talc | 0.18 |
| Carnauba Wax | 0.15 |
| | 100 |

### Syrup 1

| Item name | Pct(%) |
|---|---|
| Xylitol | 68 |
| Acacia solution 35% | 16 |
| WATER | 12 |
| Mannitol | 4 |
| Total | 100 |

The chewing gums were formulated into 2.0-2.2 g rectangle shaped gums.

### Example 3 - Laboratory Studies of Stain Preventive Effects of Glass H in Acidic Media

In this example, the effect of tooth cleaning and stain prevention of Glass H in acidic conditions was evaluated.

### Materials and Methods

The food grade Glass-H was obtained from Fuso Refining & Processing Co Ltd (Qingdao, China). Hydroxyapatite (HAP) and citric acid were obtained from Sigma-Aldrich Company. Lipton black tea was obtained from the local grocery store.

### Pre-treatment of HAP powder with Glass H

For testing the staining preventive effect of Glass-H, 200mg of HAP was added to 50mL Falcon centrifuge tubes. The amount of HAP creates a surface area that is comparable to the tooth surface area in human mouth. Then 100mg, 50mg, or 25mg or no Glass-H was added to each of the falcon tube containing 200mg of HAP to create a mixture containing 0.5%, 0.25%, 0.12% or 0% of Glass-H. 20mL of D.I. water was added and was vigorously vortexed for 1min for the pre-treatment. All test tubes were centrifuged at 5000 rpm for 5min to separate the HAP from solution. The HAP pellets treated by different Glass-H concentrations were collected.

To test the effect of Glass-H in the presence of acid, 0.12g of citric acid was added to each of the test tube and all the pre-treatment described above was repeated. This created a concentration of 0.6% citric acid, the typical level of fruity acid in saliva when consumers chew sugar free gum containing 1-2% of acid. All test tubes were centrifuged for 5min at 5000rpm. HAP pellets treated by (or non-treated) were separated from the supernatants.

### Tea Staining

250mL of water was heated by a microwave to boil. 4 bags of Lipton black tea were added to the boiled water and were swirled to create Lipton tea infusion. It was cooled to room temperature. 20mL of black tea infusion was added to each of the test tube containing the HAP pellet pre-treated by Glass-H. The test tube was shaken briefly to redisperse the pellet. It was then vortexed vigorously for 1min for tea staining. Finally, the Falcon tube was centrifuged at 5000rpm for 5min to separate HAP from the tea infusion. One of the test tubes with 200mg of HAP was treated by 20mL of D.I. water and was used as an un-stained control.

### Testing for tea staining

All tea-stained, or water washed HAP pellets were separated from the supernatants. The CIE color index L*, a* and b* of the HAP pellet was measured by a handheld colorimeter (Minolta Model CM-2600d). Whiteness index, defined by: $WI = 100 - \sqrt{{\left(L∗-100\right)}^{2} + a {∗}^{2} + b {∗}^{2}}$ *,* was calculated. In addition, the HAP pellet was transferred to a cardboard paper, and photograph was taken by a video camera.

### Testing Results

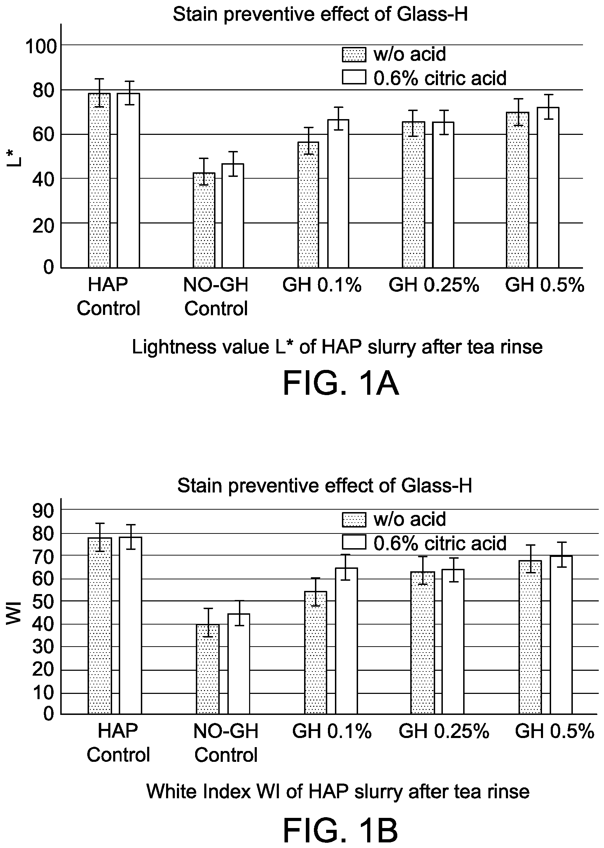

Figs. 1A and 1B show both lightness value L* (Fig. 1A), and whiteness index WI (Fig. 1B) of hydroxyaptitte powder after staining by Lipton tea. It is seen that the L* value or WI of HAP pellet changed from about 80 to about 45 after tea stain without pre-treatment of Glass-H. Pre-treatment of HAP by different levels of Glass-H, namely, 0.2%, 0.25% and 0.5%, was able to maintain WI closer to 80. It was observed that presence of 0.6% of citric acid does not impact the preventive effect of tea stain.

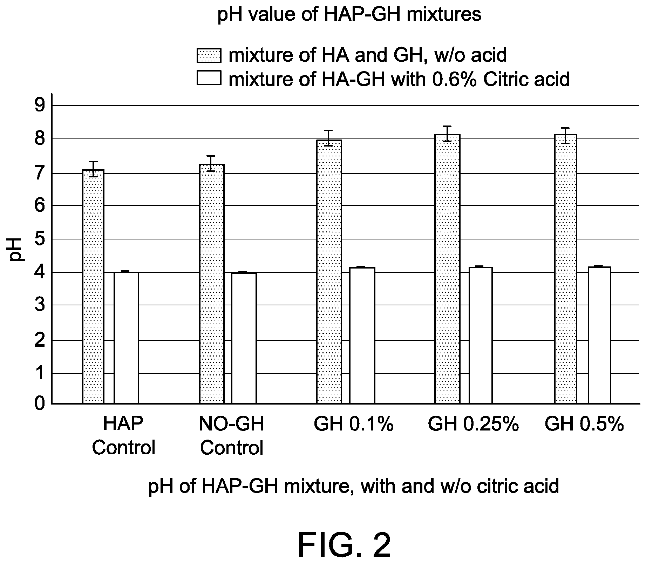

Fig. 2 shows the pH value of of the HAP solution after mixing with different level of Glass-H with and w/o the citric acid. It was observed that the mixture of Glass-H and HAP had a pH of 8.10. Adding 0.6% of citric acid to the solution reduced pH to about 4.0. These results show that change of pH doesn't alter the stain perventive effect of Glass-H.

### Discussion and Conclusion

Tea, coffee and red wines are common food drinks that are accepted by global consumers. Among these common food drinks, black tea was found readily to introduce tooth stain and cause tooth coloration overtime due to presence of high level of polyphenols. It was observed that stains formed by tea polyphenols are very difficult to remove due to its high affinity to dental or ceramic substrates.

In this laboratory test, we applied black tea as a model drink to stain the HAP powder, and measured the cleanliness or stain preventive effect of Glass-H. The cleaning or preventive effect is applicable to the other food staining as well. We observed that tea stains are difficult to remove by water. Nevertheless, the HAP powder pre-treated by Glass-H at different concentrations can effectively reduce the tea stain formation. Moreover, this preventive effect is not influenced by the presence of citric acid at pH 4.0. This example shows that this shielding and protecting effect was well maintained at acidic media with a low pH.

### Example 4 - Chewing Machine Study of Sugar Free Gum Containing a Low Level of Glass H for Inhibition of Stain Formation

This study evaluated whether a low level, 0.5% of Glass-H in sugar free gum, can effectively prevent tooth stain formation from tea and oral bacteria by a chewing machine test. The study also evaluated if the low level of GH-gum with different serving size (1, 1.5 and 2 pcs per serving) performed equivalently vs 1 piece of sugar free gum with 2% of Glass-H.

### Formulation of sugar-free soft chewing gum

Soft chew pellet gum with and without Glass-H were formulated. All soft chew gum contained 80% of gum center and 20% of coating, with piece weight of 2.2 grams. Table J shows the detailed formulations of the center layer of sugar free gum (SFG). In order to test the different serving size of SFG, we requested the testing site to cut the SFG to half (weigh up to 0.1g accuracy) and add to 1 more piece, or to combine 2 pcs of SFG together. Tables J1 and J2 list the details of testing articles.

**Table J1 - Testing articles for the chewing machine study**

| **No** | **Sample** | **Form** | **Pcs** | Piece wt | **Glass-H** |
|---|---|---|---|---|---|
| **1** | Control-1 | Soft-chew (w/o Glass H) | 1 piece | 2.2g | 0 |
| **2** | Test-1 | Soft-chew with 0.5% Glass H | 1 piece | 2.2g | 11mg |
| **3** | Test-3 | Soft-chew with 1.94% Glass H in center layer | 1 piece | 2.2g | 35.2mg |
| **4** | Control-2 | Soft-chew (w/o Glass H) | 1.5pcs | 3.3g | 0 |
| 5 | Test-4 | Soft-chew with 0.5% Glass H | 1.5pcs | 3.3g | 16.5mg |
| 6 | Test-5 | Soft-chew with 0.5% Glass H | 2pcs | 4.4g | 22mg |

**Table J2 - Soft chew gum center formula**

| | No-GH Control | 0.5% GH | 2% GH |
|---|---|---|---|
| Ingredient | % | % | % |
| Xylitol | 58.620 | 57.995 | 56.620 |
| Gumbase | 15.600 | 15.600 | 15.600 |
| Gumbase NON GMO | 10.400 | 10.400 | 10.400 |
| Sorbitol | 12.000 | 12.000 | 12.000 |
| HIS | 1.300 | 1.300 | 1.300 |
| Peppermint | 1.230 | 1.230 | 1.230 |
| Menthol | 0.400 | 0.400 | 0.400 |
| MCT | 0.250 | 0.250 | 0.250 |
| Cooling agent | 0.100 | 0.100 | 0.100 |
| HIS | 0.100 | 0.100 | 0.100 |
| Colorant | 0.000 | 0.000 | 0.000 |
| Glass H | 0.000 | 0.625 | 2.000 |
| Total | 100.000 | 100.000 | 100.000 |

### Chewing Machine Test

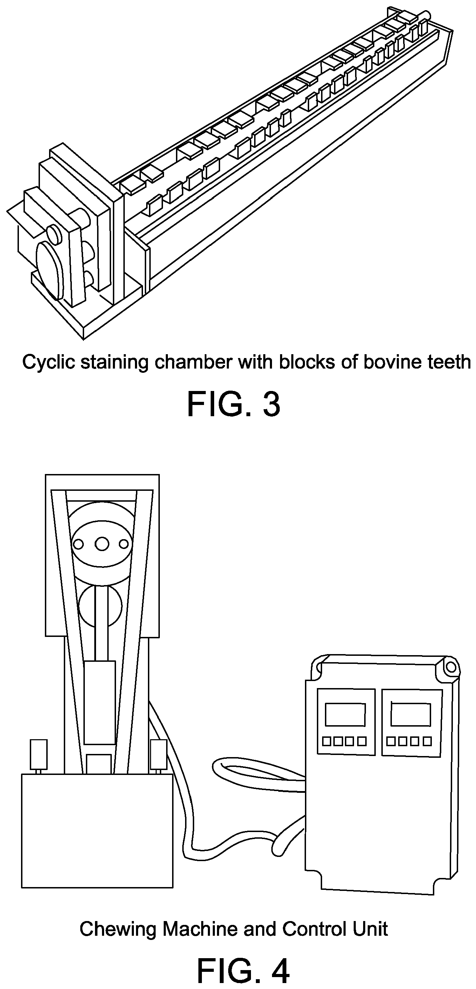

The chewing machine equipment in this test was provided with two rotary arms and two bovine teeth blocks with a flow cell at the bottom (Fig. 4). During the study, bovine teeth blocks were continuously stained in a staining cyclic chamber consists of tea, coffee, mucin and artificial saliva for 14 days (Fig. 3). On the testing day, the experimental gum was loaded to the chewing machine, and mechanically chewed 3x per day, 12 minutes each time. At 7 days and 14 days, the extent of stain was evaluated by a portable colorimeter.

### Testing Results

Table K shows the mean L*, a* and b* values of bovine tooth blocks at T0, 7-day and 14-day treatment. Table L shows the mean difference of L*, a* and b* from T0 after treatment of 7 and 14 days. We found that the lightness value, L*, changed from 70.66 to 44.26 and 40.55 at 0, 7, & 14 days, respectively, for 1 piece of soft chew gum w/o Glass-H. Bovine teeth chewed by SFG containing 0.5% or 2% Glass-H showed a less reduction of L*, indicating a stain inhibitive effect of Glass-H.

To eliminate the color difference between different tooth block, we calculated the difference of L*, a* and b* values from baseline, and performed pairwise t-test between different testing groups. Tables M-O show 2 side p values for _{ΔL}* value. The pairwise t-test analysis indicated that either 1.5pcs or 2pcs of 0.5% GH-gum performed significantly better than either 1 piece or 1.5pcs of No-GH gum controls at both 7 and 14 days (2-side p<0.05) (See Tables M-O). On the other hand, 1 piece of SFG with 0.5% GH showed statistically no different from 1 piece of No-gum control. This suggested a minimum dose requirement for Glass-H in SFG: below this level, GH showed no difference vs the control gum. Above the level, it showed a significant preventive effect. The minimum effective level of Glass-H seems about 16.5mg per serving, i.e., 1.5 pcs of SFG containing 0.5% of GH. (Last column in Table J). Statistical analysis also showed that both 1.5pcs and 2pcs of 0.5% GH-gums performed equivalently (or no difference) vs. 1 piece of 2% GH-gum (2-side p>0.5).

To further evaluate the stain preventive effect, we calculated the whiteness index of each tooth block using the following formula (Moore et al, In vitro tooth whitening effect of two medicated chewing gums compared to a whitening gum and saliva, BMC, Oral Health 8:23, 2008): $WI = 100 - \sqrt{{\left(L∗-100\right)}^{2} + a {∗}^{2} + b {∗}^{2}} ,$ WI = 100 for a perfect white block, and WI = 0 for a perfect black block.

The difference of whiteness index from baseline for each tooth block was calculated to eliminate the initial color variation of different tooth blocks: $ΔW = {WI}_{baseline} - {WI}_{after treatment}$

We performed pairwise t-test for ΔW values for all testing articles at 7 and 14 days. Tables Q and R list the 2-side p values. We observed that chewing 1.5 or 2pcs of 0.5% GH gum had a significant prevention of stain formation vs no-GH gum controls for both 7 and 14 days (2-side p< 0.05). Finally, mechanical chewing of 1.5 or 2 pcs of GH-gum showed no difference from 1 piece of 2% GH-gum for both 7 and 14 days (2-side p>0.5).

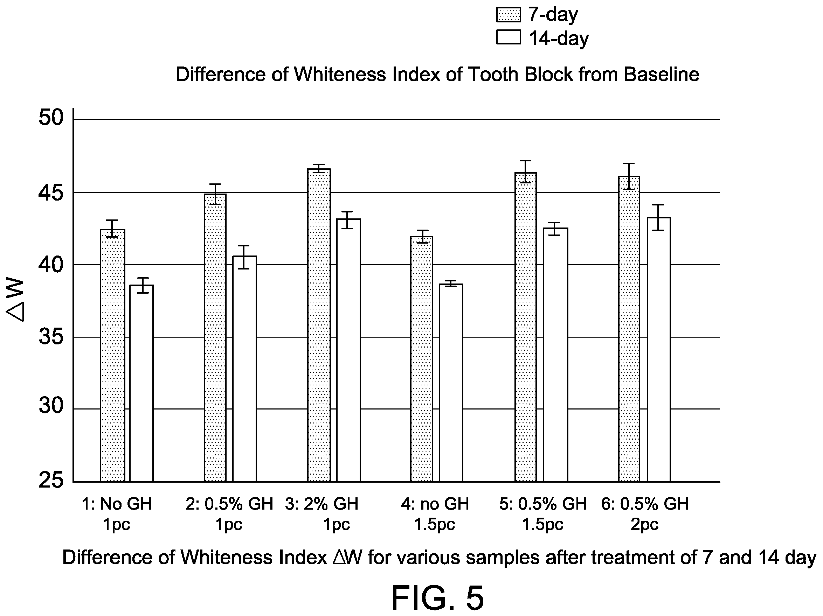

Fig. 5 shows the difference of WI from baseline for different samples after treatment for 7 and 14 days.

**Table K - average C.I.E Color Index of Bovine Tooth Blocks at Baseline (T0), 7 and 14 days**

| | | Baseline | | | 7 Days | | | 14 Days | | |
|---|---|---|---|---|---|---|---|---|---|---|
| Group | Sample | L* | a* | b* | L* | a* | b* | L* | a* | b* |
| 1 | NO Glass H, 1 piece | 70.66 | 0.61 | 2.33 | 44.26 | 4.66 | 13.26 | 40.55 | 5.75 | 14.50 |
| 2 | 0.5% Glass H 1 piece | 71.23 | 0.78 | 2.31 | 46.51 | 4.45 | 12.57 | 42.49 | 5.40 | 14.19 |
| 3 | 2.0% Glass H 1 piece | 70.95 | 0.66 | 3.33 | 48.27 | 3.69 | 12.38 | 45.04 | 4.54 | 13.95 |
| 4 | NO Glass H 1.5pcs | 70.76 | 0.55 | 3.61 | 43.83 | 4.98 | 13.41 | 40.36 | 5.71 | 12.99 |
| 5 | 0.5% Glass H 1.5pcs | 70.23 | 0.24 | 2.34 | 48.20 | 4.02 | 12.87 | 44.55 | 5.04 | 14.28 |
| 6 | 0.5% Glass H 2pcs | 71.21 | 0.92 | 3.12 | 47.85 | 4.34 | 12.86 | 45.22 | 5.00 | 13.74 |

**Table L - Difference of L*, a* and b* from baseline of bovine tooth blocks at 7 and 14 days**

| | | 7-Day Difference | | | | 14-Day Difference | | | |
|---|---|---|---|---|---|---|---|---|---|
| Group | Ingredient | ΔL* | Δa* | Δb* | ΔE | ΔL* | Δa* | Δb* | ΔE |
| 1 | NO Glass H 1 piece | -26.40 | 4.05 | 10.94 | 28.91 | -30.11 | 5.14 | 12.17 | 32.91 |
| 2 | 0.5% Glass H 1 piece | -24.71 | 3.67 | 10.26 | 27.02 | -28.74 | 4.62 | 11.88 | 31.45 |
| 3 | 2.0% Glass H 1 piece | -22.68 | 3.03 | 9.05 | 24.72 | -25.91 | 3.88 | 10.62 | 28.34 |
| 4 | NO Glass H 1.5pc | -26.94 | 4.36 | 9.81 | 29.06 | -30.40 | 5.09 | 9.39 | 32.29 |
| 5 | 0.5% Glass H 1.5pc | -22.03 | 3.79 | 10.53 | 24.72 | -25.67 | 4.81 | 11.94 | 28.73 |
| 6 | 0.5% Glass H 2pc | -23.36 | 3.56 | 9.75 | 25.58 | -25.99 | 4.22 | 10.63 | 28.41 |

**Table M - Pairwise p value for L* tooth blocks at T0 (baseline)**

| | No GH,1 piece | No GH, 1.5pcs | 0.5% GH, 1 piece | 0.5% GH, 1.5pcs | 0.5% GH, 2pcs | 2% GH, 1 piece |
|---|---|---|---|---|---|---|
| No GH,1 piece | --- | 0.933 | 0.615 | 0.933 | 0.788 | 0.845 |
| No GH, 1.5pcs | | --- | 0.750 | 0.371 | 0.817 | 0.884 |
| 0.5% GH, 1 piece | | | --- | 0.365 | 0.993 | 0.777 |
| 0.5% GH, 1.5pcs | | | | --- | 0.652 | 0.447 |
| 0.5% GH, 2pcs | | | | | --- | 0.928 |
| 2% GH, 1 piece | | | | | | --- |

**Table N - Pairwise p value for ΔL* at 7 days**

| | No GH,1 piece | No GH, 1.5pcs | 0.5% GH, 1 piece | 0.5% GH, 1.5pcs | 0.5% GH, 2pcs | 2% GH, 1 piece |
|---|---|---|---|---|---|---|
| No GH,1 piece | --- | 0.236 | 0.339 | **0.013** | **0.031** | 0.064 |
| No GH, 1.5pcs | | --- | 0.181 | **0.003** | **0.036** | **0.025** |
| 0.5% GH, 1 piece | | | --- | 0.182 | 0.514 | **0.022** |
| 0.5% GH, 1.5pcs | | | | --- | 0.409 | 0.635 |
| 0.5% GH, 2pcs | | | | | --- | 0.700 |
| 2% GH, 1 piece | | | | | | --- |

**Table O - Pairwise p value for ΔL* at 14 days**

| | No GH,1 piece | No GH, 1.5pcs | 0.5% GH, 1 piece | 0.5% GH, 1.5pcs | 0.5% GH, 2pcs | 2% GH, 1 piece |
|---|---|---|---|---|---|---|
| No GH,1 piece | --- | 0.703 | 0.431 | **0.003** | **0.017** | **0.058** |
| No GH, 1.5pcs | | --- | 0.256 | **0.000** | **0.013** | **0.027** |
| 0.5% GH, 1 piece | | | --- | 0.102 | 0.214 | **0.009** |
| 0.5% GH, 1.5pcs | | | | --- | 0.720 | 0.859 |
| 0.5% GH, 2pcs | | | | | --- | 0.962 |
| 2% GH, 1 piece | | | | | | --- |

**Table P - WI and standard deviation of bovine tooth blocks**

| | WI | | | Standard deviation | | |
|---|---|---|---|---|---|---|
| | Baseline | 7 Days | 14 Days | Baseline | 7 Days | 14 Days |
| 1: No GH 1 piece | 70.51 | 42.51 | 38.53 | 0.85 | 0.57 | 0.51 |
| 2: 0.5% GH 1 piece | 71.11 | 44.87 | 40.51 | 1.24 | 0.72 | 0.79 |
| 3: 2% GH 1 piece | 70.72 | 46.68 | 43.10 | 1.10 | 0.32 | 0.60 |
| 4: No GH 1.5pc | 70.46 | 42.03 | 38.69 | 0.34 | 0.42 | 0.15 |
| 5: 0.5% GH 1.5pc | 70.11 | 46.47 | 42.52 | 0.38 | 0.75 | 0.44 |
| 6: 0.5% GH 2pc | 71.01 | 46.10 | 43.29 | 1.76 | 0.86 | 0.87 |

**Table Q - Pairwise 2-side p value for ΔW at 7 days**

| | 1: No GH 1 piece | 2: 0.5% GH 1 piece | 3: 2% GH 1 piece | 4: No GH 1.5p | 5: 0.5% GH 1.5p | 6: 0.5% GH 2p |
|---|---|---|---|---|---|---|
| 1: No GH 1 piece | --- | 0.358 | **0.043** | 0.465 | **0.020** | **0.032** |
| 2: 0.5% GH 1 piece | | --- | **0.035** | 0.187 | 0.208 | 0.560 |
| 3: 2% GH 1 piece | | | --- | 0.010 | **0.742** | **0.623** |
| 4: No GH 1.5pcs | | | | --- | **0.005** | **0.048** |
| 5: 0.5% GH 1.5pcs | | | | | --- | 0.480 |
| 6: 0.5% GH 2pcs | | | | | | --- |

**Table R - Pairwise2-side P value for ΔW at 14 days**

| | 1: No GH 1 piece | 2: 0.5% GH 1 piece | 3: 2% GH 1 piece | 4: No GH 1.5p | 5: 0.5% GH 1.5p | 6: 0.5% GH 2p |
|---|---|---|---|---|---|---|
| 1: No GH 1 piece | --- | 0.457 | 0.048 | 0.819 | 0.007 | 0.016 |
| 2: 0.5% GH 1 piece | | --- | 0.017 | 0.425 | 0.109 | 0.236 |
| 3: 2% GH 1 piece | | | --- | 0.027 | 0.983 | 0.953 |
| 4: No GH 1.5p | | | | --- | 0.001 | 0.018 |
| 5: 0.5% GH 1.5p | | | | | --- | 0.909 |
| 6: 0.5% GH 2p | | | | | | --- |

### Conclusion

The chewing machine test demonstrated that both 1.5 and 2 pieces of SFG with 0.5% of Glass-H were able to perform significantly better than the control gums without Glass-H (either 1 piece or 1.5pcs). They showed no difference vs 1 piece of SFG with 2% Glass-H for prevention of stain formation. The study suggested a minimum level of 16.5mg Glass-H per serving.

### Example 5 - Stability of Glass-H Gum

### Background

Glass-H shows a poor stability in aqueous media, is highly moisture sensitive, and the degradation of Glass-H is accelerated in acidic environment. In Chapter 8 in the book of "Phosphorus and Its Compounds", published in 1958, John Wazer reported that the hydrolytic degradation of condensed polyphosphates is strongly catalyzed by acid. For example, the rate of degradation increases over 10x when pH changes from 6 to 4. For this reason, it was recommended that Glass-H has to be formulated an oral care product with a low moisture level at neutral to basic pH. Nevertheless, there is a strong consumer desire for sugar-free chewing gum with an enjoyable fruity flavor meanwhile helps maintain good oral health condition.

Acids such as citric acid, malic acid and lactic acid, are often incorporated in the fruity confections for the desired sensory taste. In this example, Glass-H was formulated with various fruity flavors, including lime, citrus, and watermelon with 0.5-2% of fruity acids. To ensure the effect of tooth cleaning and stain prevention of Glass-H in the acidic condition, a laboratory test was performed to evaluate the stability of Glass-H gum at accelerated testing conditions, measured by a commercially available kit for orthophosphate and total phosphate.

### Degradation of Glass-H

Glass-H is a condensed phosphate polymer linked by pyrophosphate unit (P-OP). It consists of a mixture of linear polyphosphate polymers with an average phosphate chain length varies from 6 to 21. In the presence of moisture, Glass-H degrades very quickly and forms the low molecular weight polyphosphates, eventually forms ortho-phosphate. The degradation of Glass-H is accelerated at high temperate and at high concentration. It is further accelerated in the presence of organic fruity acids.

Glass-H can be analyzed by an ion chromatograph (IC) measurement. Alternative, one can analyze the concentration of mono-phosphate ions, commonly known as ortho-phosphate, in combination with the total polyphosphate ions (including mono-phosphate) to assay the Glass-H: $\begin{matrix}Glass - H level = total polyphosphate level \left(including orthophosphate\right) - orthophosphate \\ level\end{matrix}$

There are many commercial testing kits available for quantitative analysis of orthophosphate and total polyphosphate ions.

### Determination of the stability of sugar free gum (SFG) containing 0.5% of Glass-H at accelerated condition

For determination of the stability of sugar free gum (SFG) with Glass-H, food grade Glass-H was obtained from Fuso Refining & Processing Co Ltd (Qingdao, China) (Glass-H CN), and from BK Giulini GmbH, Werk Ladenburg, Germany (Glass-H DE). The Glass-H CN has an average chain length of 9, while Glass-H DE has a chain length of 20. The Glass-H was formulated in sugar-free chewing gum at 0.5% level according to the previous examples, together with fruit acids and/or without acids.

To test the stability, products were packed in a plastic package and placed at 45°C and 85% humidity controlled chamber (RH) for 7 and 14 days. After 7 and 14 days, the chewing gum samples were pulled out of 45°C/85% RH humidity chamber. They were visually examined for color, integrity, free-flow characters. They were also tasted for sensory evaluation. Table S below shows the characteristics of chewing gum samples.

**Table S - Characteristics of Glass-H gum after exposure to 45°C/85% RH humidity controlled chamber**

| **Sample** | **Duration** | **Color** | **Free-flow** | **Integrity** | **Taste/sensory** |
|---|---|---|---|---|---|
| Sugar-free gum with 0.5% Glass-H CN | 7-day | No change | Yes | OK | Pass |
| | 14-day | Slightly brownish | Yes | OK | Pass |
| Sugar-free gum with fruit acids and 0.5% Glass-H CN | 7-day | No change | Yes | OK | Pass |
| | 14-day | Slightly brownish | Yes | OK | Pass |
| Sugar-free gum with fruit acids and 0.5% Glass-H DE | 7-day | No change | Yes | OK | Pass |
| | 14-day | Slightly brownish | Yes | OK | Pass |

For analysis of degradation, all Glass-H was extracted by dissolving the chewing gum in a mixture of toluene- non-phosphate-pH7.0 buffer. 1mL of aqueous solution was tested by a MQUANT Phosphate Test kits available from Fisher-scientific Company (Catalog No. M1104280001). This test kits provide assay of orthro-phosphate at range between 10-500mg/L, or 10-500ppm. Prior to the test, various commercial phosphate salts were analyzed, including sodium phosphate, sodium pyrophosphate, long chain and short chain Glass-H, and a good result was obtained, including accuracy and sensitivity (up to 10ppm).

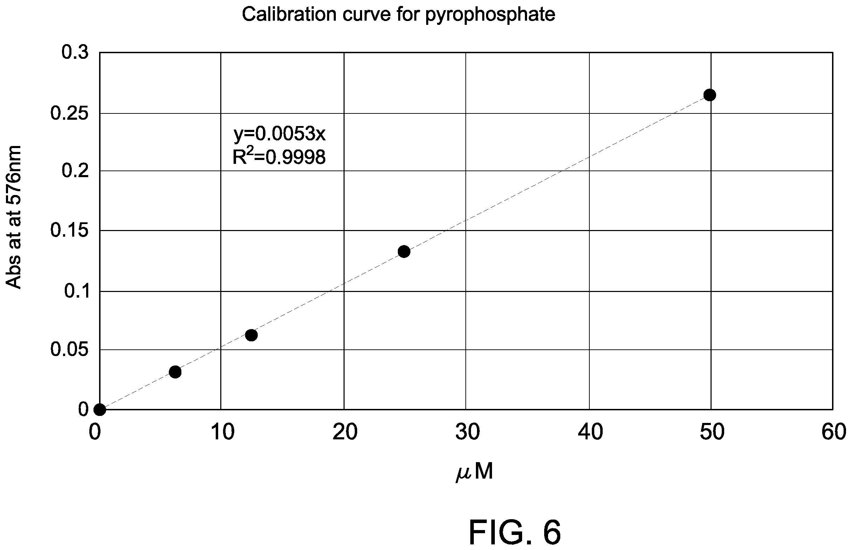

For analysis of total polyphosphate (including orthophosphate), an Invitrogen PiPer Phosphate Assay Kit (Cat No. P22062) was used. The kit contained a pyrophosphatase enzyme that converts all condensed polyphosphate to ortho-phosphate ion, which reacts with maltose to form glucose that triggers a cascade of reactions. It forms Amplex Red, which can be measured by a spectrophotometer. The PiPer Kit provides an ultrasensitive way to measure total phosphate ions, with a detecting limit close to 5µM or 1.3ppm. Fig. 6 shows the calibration curve for a quantitative analysis of polyphosphate by a Beckman spectrophotometer at 576nm (Beckman Coulter Model DU Series 700).

Table T shows the mg of total sodium phosphate, sodium orthro phosphate and Glass-H level in the chewing gum. Very little degradation of Glass-H was observed for all sugar free gum tested, with presence of fruit acids and w/o acids.

**Table T - Glass-H degradation after 2-week exposure at 45°C/85%RH humidity-controlled chamber**

| **Sample** | **Duration at 45°C/85%RH** | **Total Phosphate** | **Ortho-phosphate** | **Glass-H** | **Degradation** |
|---|---|---|---|---|---|
| SFG with 0.5% Glass-H CN | 0-day | 12.0mg | 0.25mg | 11.8mg | 2% |
| | 7-day | 12.6mg | 0.50mg | 12.1mg | 4% |
| | 14-day | 12.7mg | 0.75mg | 11.9mg | 6% |
| SFG with fruit acids and 0.5% Glass-H CN | 0-day | 12.55mg | 0.25mg | 12.6mg | 2% |
| | 7-day | 12.8mg | 0.50mg | 12.3mg | 4% |
| | 14-day | 12.2mg | 0.75mg | 11.5mg | 6% |
| SFG with fruit acids and 0.5% Glass-H DE | 0-day | 12.25 mg | 0.25mg | 12.25 | 2% |
| | 7-day | 12.0mg | 0.50mg | 11.5mg | 4% |
| | 14-day | 12.7mg | 0.50mg | 12.2mg | 4% |

There were slight degradation of Glass-H gum over 14 day exposure at 45°C/85%RH humidity-controlled chamber. Nevertheless, all samples showed less than 6% of degradation of Glass-H. These results demonstrate that chewing gum containing low level of Glass-H (at 0.5%) either formulated with fruit acids or without acids, demonstrated a good stability and product shelf-life.

This written description uses examples to disclose the invention, including the best mode, and also to enable any person skilled in the art to practice the invention, including making and using any devices or systems and performing any incorporated methods. The patentable scope of the invention is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal language of the claims.

## Claims

1. A chewing gum comprising sodium hexametaphosphate and a fruity acid, wherein the sodium hexametaphosphate is in the gum in an amount of from 0.2 wt% to less than 1 wt%, and wherein the chewing gum comprises from 0.2 to 2.0 wt% of the fruity acid.

2. The chewing gum of claim 1, comprising from 0.5 to 0.8 wt% of sodium hexametaphosphate, optionally from 0.5 to 0.7 wt%.

3. The chewing gum of claim 1, wherein the sodium hexametaphosphate is encapsulated.

4. The chewing gum of claim 1, wherein the fruity acid is selected from the group consisting of citric acid, malic acid, tartaric acid, adipic acid and combinations thereof.

5. The chewing gum of claim 1, comprising from 0.3 to 1.9 wt% of the fruity acid, optionally from 0.7 to 1.0 wt%, optionally from 0.8 to 0.9 wt%, optionally from 0.3 to 0.5 wt%.

6. The chewing gum of claim 1, wherein the fruity acid is encapsulated.

7. The chewing gum of any one of claims 1-6, wherein the gum is a center filled gum.

8. The chewing gum of claim 7 wherein the gum is a liquid center filled gum.

9. The chewing gum of claim 7, wherein the gum is a powder center filled gum.

10. The chewing gum of any one of claims 1-9, wherein the gum is coated.

11. The chewing gum of any one of claims 1-10, wherein the gum has a shape selected from the group consisting of ball, lentil, and cube.

12. The chewing gum of any one of claims 1-11, wherein the gum has a diameter of from 11 mm to 19 mm.

13. A method for the prevention of tooth staining in a consumer, the method comprising chewing a gum of any one of claims 1-12, wherein the gum provides at least 16.5 mg of sodium hexametaphosphate per serving.

14. The method of claim 13, wherein
(i) the gum is chewed for at least 10 minutes, optionally for at least 12 minutes,
or
(ii) the gum is chewed at least 1 time per day, optionally at least 2 times per day, optionally at least 3 times per day.

15. The method of claim 13, wherein the gum is chewed 1 to 3 times per day for at least 7 days, optionally chewed 1 to 3 times per day for at least 14 days.

## Patentansprüche

1. Kaugummi, aufweisend Natriumhexametaphosphat und eine fruchtige Säure, wobei das Natriumhexametaphosphat in dem Kaugummi in einer Menge von 0,2 Gew.-% bis weniger als 1 Gew.-% vorliegt und wobei der Kaugummi 0,2 bis 2,0 Gew.-% der fruchtigen Säure aufweist.

2. Kaugummi nach Anspruch 1, aufweisend 0,5 bis 0,8 Gew.-% Natriumhexametaphosphat, optional 0,5 bis 0,7 Gew.-%.

3. Kaugummi nach Anspruch 1, wobei das Natriumhexametaphosphat eingekapselt ist.

4. Kaugummi nach Anspruch 1, wobei die fruchtige Säure ausgewählt ist aus der Gruppe bestehend aus Zitronensäure, Äpfelsäure, Weinsäure, Adipinsäure und Kombinationen davon.

5. Kaugummi nach Anspruch 1, aufweisend 0,3 bis 1,9 Gew.-% der fruchtigen Säure, optional 0,7 bis 1,0 Gew.-%, optional 0,8 bis 0,9 Gew.-%, optional 0,3 bis 0,5 Gew.-%.

6. Kaugummi nach Anspruch 1, wobei die fruchtige Säure eingekapselt ist.

7. Kaugummi nach einem der Ansprüche 1 bis 6, wobei der Kaugummi ein Kaugummi mit einer Füllung in der Mitte ist.

8. Kaugummi nach Anspruch 7, wobei der Kaugummi ein Kaugummi mit einer flüssigen Füllung in der Mitte ist.

9. Kaugummi nach Anspruch 7, wobei der Kaugummi ein Kaugummi mit einer Pulverfüllung in der Mitte ist.

10. Kaugummi nach einem der Ansprüche 1 bis 9, wobei der Kaugummi beschichtet ist.

11. Kaugummi nach einem der Ansprüche 1 bis 10, wobei der Kaugummi eine Form aufweist, die aus der Gruppe bestehend aus Kugel, Linse und Würfel ausgewählt ist.

12. Kaugummi nach einem der Ansprüche 1 bis 11, wobei der Kaugummi einen Durchmesser von 11 mm bis 19 mm aufweist.

13. Verfahren zur Vorbeugung von Zahnverfärbungen bei einem Verbraucher, wobei das Verfahren das Kauen eines Kaugummis nach einem der Ansprüche 1 bis 12 aufweist, wobei der Kaugummi pro Portion mindestens 16,5 mg Natriumhexametaphosphat bereitstellt.

14. Verfahren nach Anspruch 13, wobei
(i) der Kaugummi mindestens 10 Minuten, optional mindestens 12 Minuten, gekaut wird,
oder
(ii) der Kaugummi mindestens 1-mal pro Tag, optional mindestens 2-mal pro Tag, optional mindestens 3-mal pro Tag, gekaut wird.

15. Verfahren nach Anspruch 13, wobei der Kaugummi 1- bis 3-mal pro Tag für mindestens 7 Tage, optional 1- bis 3-mal pro Tag für mindestens 14 Tage, gekaut wird.

## Revendications

1. Gomme à mâcher comprenant de l'hexamétaphosphate de sodium et un acide fruité, dans laquelle l'hexamétaphosphate de sodium est présent dans la gomme en une quantité de 0,2 % en poids à moins de 1 % en poids, et dans laquelle la gomme à mâcher comprend de 0,2 à 2,0 % en poids de l'acide fruité.

2. Gomme à mâcher selon la revendication 1, comprenant de 0,5 à 0,8 % en poids d'hexamétaphosphate de sodium, facultativement de 0,5 à 0,7 % en poids.

3. Gomme à mâcher selon la revendication 1, dans laquelle l'hexamétaphosphate de sodium est encapsulé.

4. Gomme à mâcher selon la revendication 1, dans laquelle l'acide fruité est choisi dans le groupe constitué par l'acide citrique, l'acide malique, l'acide tartrique, l'acide adipique et des combinaisons de ceux-ci.

5. Gomme à mâcher selon la revendication 1, comprenant de 0,3 à 1,9 % en poids de l'acide fruité, facultativement de 0,7 à 1,0 % en poids, facultativement de 0,8 à 0,9 % en poids, facultativement de 0,3 à 0,5 % en poids.

6. Gomme à mâcher selon la revendication 1, dans laquelle l'acide fruité est encapsulé.

7. Gomme à mâcher selon l'une quelconque des revendications 1 à 6, dans laquelle la gomme est une gomme fourrée à cœur.

8. Gomme à mâcher selon la revendication 7, dans laquelle la gomme est une gomme fourrée à cœur liquide.

9. Gomme à mâcher selon la revendication 7, dans laquelle la gomme est une gomme fourrée à cœur en poudre.

10. Gomme à mâcher selon l'une quelconque des revendications 1 à 9, dans laquelle la gomme est enrobée.

11. Gomme à mâcher selon l'une quelconque des revendications 1 à 10, dans laquelle la gomme a une forme choisie dans le groupe constitué par une boule, une lentille et un cube.

12. Gomme à mâcher selon l'une quelconque des revendications 1 à 11, dans laquelle la gomme a un diamètre de 11 mm à 19 mm.

13. Procédé pour la prévention de la coloration des dents chez un consommateur, le procédé comprenant la mastication d'une gomme selon l'une quelconque des revendications 1 à 12, dans lequel la gomme fournit au moins 16,5 mg d'hexamétaphosphate de sodium par portion.

14. Procédé selon la revendication 13, dans lequel
(i) la gomme est mâchée pendant au moins 10 minutes, facultativement pendant au moins 12 minutes,
ou
(ii) la gomme est mâchée au moins 1 fois par jour, facultativement au moins 2 fois par jour, facultativement au moins 3 fois par jour.

15. Procédé selon la revendication 13, dans lequel la gomme est mâchée 1 à 3 fois par jour pendant au moins 7 jours, facultativement mâchée 1 à 3 fois par jour pendant au moins 14 jours.
